# EUROPÄISCHE PATENTSCHRIFT

(11) **EP 0 444 103 B1**
(45) Veröffentlichungstag und Bekanntmachung des Hinweises auf die Patenterteilung: **12.09.2001**
(21) Anmeldenummer: 89913071.0
(22) Anmeldetag: 15.11.1989
(51) Int. Cl.: C09B 61/00, A23J 1/04

(54) **VERFAHREN ZUR HERSTELLUNG VON ASTAXANTHIN UND VERWANDTEN CAROTENOIDEN, ASTAXANTHINESTERN, CHITIN, PROTEINEN UND FLEISCH AUS PFLANZEN, ALGEN, BAKTERIEN, KRILL, GARNELEN, LANGUSTEN UND ANDEREN KREBSTIEREN**
A PROCEDURE FOR THE PRODUCTION OF ASTAXANTHIN AND RELATED CAROTENOIDS, ASTAXANTHIN ESTERS, CHITIN, PROTEINS AND MEAT FROM PLANTS, ALGAE, BACTERIA, KRILL, SHRIMPS AND OTHER CRAYFISH AND CRUSTACEA
PROCEDE DE PRODUCTION D'ASTAXANTHINE ET DE CAROTENOIDES ASSOCIES, D'ESTERS D'ASTAXANTHINE, DE CHITINE, DE PROTEINES ET DE CHAIRS A PARTIR DE PLANTES, D'ALGUES, DE BACTERIES, DE KRILLES, DE CREVETTES ET AUTRES LANGOUSTES ET CRUSTACES

(30) Priorität: 18.11.1988 NO 885150
(43) Veröffentlichungstag der Anmeldung: 04.09.1991
(73) Patentinhaber: MIKALSEN, Ester, N-6100 Volda (NO)
(72) Erfinder: MIKALSEN, Gunnar, deceased (NO)
(74) Vertreter: TER MEER STEINMEISTER & PARTNER GbR
(86) Internationale Anmeldenummer: NO8900119
(87) Internationale Veröffentlichungsnummer: WO9005765

(56) Entgegenhaltungen:
- GB-A- 1 224 172
- US-A- 4 505 936

## Beschreibung

Die vorliegende Erfindung betrifft ein Verfahren zum Herstellen von Astaxanthin und verwandten Carotinoiden, Astaxanthinestern, Chitin und Proteinen gemäß Anspruch 1.

Astaxanthin und verwandte Carotinoide werden heutzutage in der industriellen Fischzucht als Farbstoffe verwendet zur Farbgebung des Fischfleisches von Arten, die in natürlicher Umgebung rotes oder hellrotes Fleisch haben. Während der Aufzucht gibt es kein Futter, das genug Farbstoff enthält, um solchem Fisch derart farbiges Fleisch zu geben. Diese Farbstoffe werden heutzutage durch Fermentieren von Algen und Synthetisieren aus Gärungsmischungen hergestellt. Diese Vorgehensweise ist sowohl teuer als auch zeitraubend und erfordert die Verarbeitung großer Mengen an Gärungsmischungen.

In der US-A-4 505 936 wird bereits ein Verfahren zur Gewinnung von Astaxanthin aus Abfällen von Schalentieren beschrieben. Hierzu werden Abfälle von Schalentieren zunächst gemahlen oder zerrieben zum Aufreißen und Trennen des weichen proteinhaltigen Gewebes von den chitinhaltigen Schalen, wobei eine proteinhaltige Paste erhalten wird. Diese Paste wird zum Verringern des pH-Wertes mit einer Säure behandelt, um mikrobische Degeneration zu unterdrücken, den geringen in der Paste enthaltenen Anteil an chitinhaltiger Schale zu entmineralisieren und zu entkalken und die gesamte Rückgewinnung des roten Carotinoid-Pigmentes während der anschließenden Extraktion mit Öl zu verbessern. Darauf wird der angesäuerten Paste ein genießbares Öl zugemischt, worauf die ölhaltige Paste zum Optimieren der Extraktion des roten Carotinoid-Pigmentes durch das Öl über einen geeigneten Zeitraum auf eine Temperatur in der Größenordnung von 170°F bis 200°F erhitzt wird, sodann das öl-/-proteinhaltige Gemisch durch Zentrifugieren in eine Wasser-/-Öl-Phase und eine feste Phase getrennt wird, worauf die Wasser-/Öl-Phase in ein mit roten Carotinoid-Pigmenten angereichertes Öl und einen aromatischen Wasserextrakt getrennt wird. - Zum Durchführen dieses Verfahrens ist ein umfangreicher und kostspieliger apparativer Aufwand erforderlich, zudem ist das Verfahren umständlich und schon wegen der Notwendigkeit des Einsatzes von Speiseöl wenig effizient.

Es ist bisher kein Verfahren bekannt, welches der Notwendigkeit eines kostengünstigen industriellen Prozesses für die Herstellung von Astaxanthin und verwandten Carotinoiden in der industriellen Fischzucht und anderen Anwendungen für die Farbgebung von Nahrungsmitteln gerecht wird. Die vorliegende Erfindung beschreibt ein Verfahren, welches ermöglicht, Astaxanthin und verwandte Carotinoide viel billiger als mit der heutigen Technologie auf industrieller Grundlage herzustellen. Gleichzeitig gründet sich die Erfindung auf die Ausnutzung natürlicher Vorkommen, die (bisher) unverwertbar sind oder die ein Abfallbeseitigungsproblem darstellen. Dies schließt antarktischen Krill ein; Pflanzen, Bakterien und Algen; und Abfall aus der gewerblichen Verarbeitung von Fleisch solcher Arten wie Garnelen, Krebsen (Langusten) und Krustentieren. Die vorliegende Erfindung hilft auch, die oben erwähnten natürlichen Vorkommen besser auszunutzen, indem ein größerer Anteil des Rohstoffes verwendet wird, entweder durch Verbesserung des Ausnutzungsgrades während der Herstellung der gewünschten Produkte oder durch Verwendung desselben Rohstoffes zum Herstellen anderer Arten von Produkten. So kann die vorliegende Erfindung beispielsweise eingesetzt werden zur Herstellung von Astaxanthin und verwandten Carotinoiden, Astaxanthinestern, Chitin, Proteinen und Fleisch aus antarktischem Krill auf eine Weise, die einen extrem hohen Ausnutzungsgrad des Rohstoffes zuläßt.

Die Schalen von antarktischem Krill stellen darüberhinaus die größte ungenutzte Quelle von Protein in der Welt dar. Jedoch können sie nicht für Futter- oder Nahrungszwecke verwendet werden, weil sie zu viele toxische Bestandteile wie Fluor und toxische Schwermetalle enthalten. Mit den Herstellungsmitteln nach der vorliegenden Erfindung können Fluor und toxische Schwermetalle aus den Schalen entfernt werden, so daß weitgehender Gebrauch von den aus ihnen hergestellten Proteinen und Chitin gemacht werden kann.

Diese und andere Merkmale der Erfindung sind dem kennzeichnenden Teil des untenstehenden Patentanspruches zu entnehmen.

Das Verfahren nach dieser Erfindung wird nun durch die folgenden Beispiele mit weiteren Einzelheiten erläutert.

### Beispiel 1, Krill

Nachdem der antarktische Krill gefangen ist, wird das hinter dem Cephalothorax sitzende Fleisch ausgepreßt unter Benutzung entsprechend geänderter, für die Verarbeitung von Garnelen vorgesehener Einrichtungen.

Diese Extraktion des Fleisches muß innerhalb von drei bis vier Stunden nach dem Fang vorgenommen werden, um die Verseuchungen durch die Schalen, den Kopf und den Cephalothorax (Mittelleib) zu verhindern, die das Fleisch beeinträchtigen könnten. Da außerdem die Autolyse von Krill sehr schnell und leicht geschieht, ist dies ein weiterer Grund, warum das Fleisch innerhalb von vier Stunden nach dem Fang extrahiert werden muß. Nachdem das Fleisch ausgepreßt worden ist, muß es bei einer Temperatur unterhalb von minus 40 Grad Celsius gelagert werden, bis es verarbeitet wird. Die Verseuchungsstoffe in Krill sind hauptsächlich Fluor in der Schale sowie toxische Schwermetalle wie Cadmium, Quecksilber, Blei und Zink. Es gibt hohe Konzentrationen von Cadmium im Cephalothorax und dem Inhalt des Kopfes.

Nachdem das Fleisch extrahiert worden ist, kann die weitere Behandlung des Restes des Krills, z.B. der Schale, des Cephalothorax und des Kopfes mit seinem Inhalt, hier zusammenfassend als Krillschale bezeichnet, auf unterschiedliche Weise vorgenommen werden.

### Alternative A:

Die Krillschale wird in 1.0 N NaOH 30 Minuten lang gekocht. Dann wird der Extrakt vom Rückstand getrennt. Der Rückstand wird dann wiederum in 1.0 N NaOH 30 Minuten lang gekocht und der Extrakt wird ein weiteres Mal vom Rückstand getrennt. Der Rückstand wird sodann in konzentrierter Ameisensäure getrocknet, bevor er in Anhydro-Ameisensäure oder einer anderen stark konzentrierten Säure gelöst und filtriert wird. Dann wird dem Filtrat Wasser zugesetzt zum Ausfällen des gereinigten Chitins. Die Extrakte aus den beiden Kochvorgängen werden gemischt und können entweder:
a: eine Säure zugesetzt erhalten bis alles Astaxanthin und verwandte Carotinoide, Astaxanthinester und Proteine ausgefällt sind, wonach das Fällungsprodukt durch Filtrieren oder einen ähnlichen Vorgang aus der Lösung getrennt wird; oder
b: können zum Abkühlen entnommen werden, wonach die Mischung in drei Phasen getrennt wird welche aufgeteilt werden durch Abschwemmen der Phase mit dem niedrigsten spezifischen Gewicht und Dekantieren (oder Gleichwertiges) der Phase mit dem dazwischenliegenden spezifischen Gewicht von der Phase mit dem höchsten spezifischen Gewicht; wobei die Phase mit dem niedrigsten spezifischen Gewicht durch Zusatz einer Säure neutralisiert wird, was die Bildung zweier flüssiger Phasen bewirkt, die voneinander getrennt werden und die aus Astaxanthinestern bestehende ölige Phase wird damit erhalten; wobei die Phase mit dem höchsten spezifischen Gewicht durch eine Säure neutralisiert wird und die aus Astaxanthin und verwandten Carotinoiden bestehenden Feststoffe werden ausgefiltert und damit erhalten; und wobei die Phase mit dem dazwischenliegenden spezifischen Gewicht neutralisiert wird und erhält Fischtran (Fischöl), Ca²⁺-Ionen, S²⁻-Ionen und (NH₄)₂HPO₄ für die Ausfällung von Fluor und Schwermetallverseuchungen, wobei dieses Fällungsprodukt durch Abschwemmen oder Gleichwertiges aus der Lösung entfernt wird. Danach wird diese Lösung vor der Ausfällung des gereinigten Proteins angesäuert, gefolgt von der Trennung dieser Proteine von der Lösung durch Filtrieren oder eine gleichwertige Methode.

### Alternative B:

Die Krillschale wird 30 Minuten lang in 0.01 N NaOH gekocht. Dann wird der Extrakt vom Rückstand getrennt. Der Rückstand wird dann erneut in 0.01 N NaOH 30 Minuten lang gekocht und der Extrakt wird ein weiteres Mal vom Rückstand getrennt. Dann wird der Rückstand wiederum und dieses Mal in 1.0 N NaOH 30 Minuten lang gekocht und der Extrakt wird wieder vom Rückstand getrennt. Der Rückstand wird in konzentrierter Ameisensäure getrocknet, bevor er in Anhydro-Ameisensäure oder einer anderen starken konzentrierten Säure gelöst und sodann gefiltert wird. Dann wird dem Filtrat wasser zugesetzt zum Ausfällen des gereinigten Chitins.

Die Extrakte der ersten beiden Kochvorgänge in 0.01 N NaOH werden gemischt, neutralisiert und es werden ihnen Fischtran, Ca²⁺-Ionen, S²⁻-Ionen und (NH₄)₂HPO₄ zugesetzt zum Ausfällen von Fluor und Schwermetallverseuchungen, wobei diese Fällungsprodukte durch Abschwemmen oder Gleichwertiges aus der Lösung entfernt werden. Hiernach wird die Lösung vor der Ausfällung des gereinigten Proteins angesäuert, gefolgt von der Trennung dieser Proteine von der Lösung durch Filtrieren oder einer gleichwertigen Methode.

Die Extrakte der letzten beiden Kochvorgänge in 1.0 N NaOH werden gemischt und können entweder:
a: eine Säure zugesetzt erhalten, bis alles Astaxanthin und verwandte Carotinoide, Astaxanthinester und Proteine ausgefällt sind, wonach das Fällungsprodukt durch Filtrieren oder einen ähnlichen Vorgang von der Lösung getrennt wird; oder
b: können zum Abkühlen abgestellt werden, wonach die Mischung in drei Phasen getrennt wird, die aufgeteilt werden durch Abschwemmen der Phase mit dem niedrigsten spezifischen Gewicht und Dekantieren (oder Gleichwertiges) der Phase mit dem dazwischenliegenden spezifischen Gewicht von der Phase mit dem höchsten spezifischen Gewicht; wobei die Phase mit dem niedrigsten spezifischen Gewicht durch Zusatz einer Säure neutralisiert wird, was die Bildung zweier flüssiger Phasen bewirkt, welche voneinander getrennt werden, und die Astaxanthinester enthaltende ölige Phase wird erhalten; wobei die Phase mit dem höchsten spezifischen Gewicht durch eine Säure neutralisiert wird und Astaxanthin und verwandte Carotinoide enthaltende Feststoffe werden ausgefiltert und damit erhalten; und wobei die Phase mit dem dazwischenliegenden spezifischen Gewicht neutralisiert wird und anschließend Fischtran, Ca²⁺-Ionen, S²⁻-Ionen und (NH₄)₂HPO₄ zugesetzt werden zum Ausfällen von Fluor und Schwermetallverseuchungen, wobei dieses Fällungsprodukt durch Abschwemmen oder Gleichwertiges aus der Lösung entfernt wird, worauf die Lösung vor der Ausfällung der gereinigten Proteine angesäuert wird mit nachfolgender Trennung dieser Proteine aus der Lösung durch Filtrieren oder eine gleichwertige Methode.

### Beispiel 2, Pflanzen, Algen und Bakterien

Astaxanthin kann mit dem in diesem Patent dargelegten Behandlungsverfahren aus Pflanzen, Algen und Bakterien hergestellt werden, die diesen Farbstoff enthalten.

Pflanzen, Algen oder Bakterien oder eine Mischung hieraus kann 30 Minuten lang gekocht werden in 1.0 N NaOH, hiernach wird der Extrakt von dem Rückstand der Pflanzen, Algen oder Bakterien separiert und der Rückstand wird zum Abkühlen entnommen. Während der Abkühlung wird das Astaxanthin separiert und schlägt sich als Sediment im Extrakt nieder. Das Sediment setzt sich zusammen aus Astaxanthin und Extrakt, wobei diese durch eine bekannte Methode voneinander getrennt werden.

### Beispiel 3, Garnelen, Krebse (Langusten) und andere Krustentiere

Garnelen, Krebse (Langusten) und Krustentiere unterscheiden sich von Krill dadurch, daß sie gekocht werden, bevor das Fleisch zum Verzehr entfernt wird. Der Rest bzw. Rückstand der Garnelen, der Krebse und Krustentiere besteht aus Schale, Kopf mit Inhalt und ungenießbaren Bestandteilen, die auf dieselbe Weise wie Krillschale verarbeitet werden können zur Herstellung von Astaxarthin und verwandten Carotinoiden, Astaxanthinestern, Proteinen und Chitin.

## Patentansprüche

1. Verfahren zum Herstellen von Astaxanthin und verwandten Carotinoiden, Astaxanthinestern, Chitin und Proteinen aus Pflanzen, Algen, Bakterien, Krill, Garnelen und anderen Krebsen und Krustentieren durch ein- oder mehrmaliges Kochen des Rohstoffes in einer Lauge mit anschließender Filtierung des Extraktes aus der kochenden Lauge, **dadurch gekennzeichnet**, daß man die Extrakte der Filtrierung sich abkühlen läßt, wobei sich die Extrakte in drei Phasen auftrennen, welche wiederum getrennt werden durch Abschwemmen der Phase mit dem niedrigsten spezifischen Gewicht und Dekantieren (oder ähnliches) der Phase mit einem dazwischenliegenden spezifischen Gewicht von der Phase mit dem höchsten spezifischen Gewicht, wobei die Phase mit dem niedrigsten spezifischen Gewicht neutralisiert wird durch Zusatz einer Säure mit dem Resultat der Bildung zweier flüssiger Phasen, welche getrennt werden, und von denen eine Astaxanthinester enthaltende ölige Phase gesammelt wird; wobei die Phase mit dem höchsten spezifischen Gewicht neutralisiert wird mit einer Säure und aus Astaxanthin und verwandten Carotinoiden bestehende Feststoffe werden herausgefiltert und gesammelt; und wobei die Phase mit dem dazwischenliegenden spezifischen Gewicht neutralisiert wird, aus welcher F⁻ -Ionen und Verseuchungsstoffe, insbesondere Schwermetallverseuchungen, entfernt werden, gefolgt von einer Ansäuerung - in einer an sich bekannten Weise - der gereinigten Phase für die Ausfällung von Proteinen, wonach die gereinigten, ausgefällten Proteine durch Filtrierung oder dgl. aus der Phase gesammelt werden; daß der Rückstand vom Kochvorgang getrocknet wird und danach in Anhydro-Ameisensäure oder einer anderen starken Säure gelöst und gefiltert wird, worauf dem Filtrat Wasser hinzugefügt wird mit dem Resultat der Ausfällung des gereinigten Chitins.

2. Verfahren nach Anspruch 1, **dadurch gekennzeichnet, daß** die Phase mit dem dazwischenliegenden spezifischen Gewicht gereinigt wird durch Zusatz eines Fischtrans (Fischöls) , Ca⁺ -Ionen, S²⁻-Ionen und (NH₄)₂HPO₄ , für die Ausfällung von etwaigen F⁻-Ionen und Verseuchungsstoffen, insbesondere Schwermetallverseuchungen, wobei diese Ausfällung durch Abschwemmen oder dgl. aus der Lösung entfernt wird.

3. Verfahren nach Anspruch 1 oder 2, **dadurch gekennzeichnet, daß** das Kochen in zwei Schritten durchgeführt wird in der Lauge, die eine Konzentration von 1.0 N einer starken alkalischen Lösung hat.

## Claims

1. Procedure for the production of astaxanthin and related carotenoids, astaxanthin esters, chitin and proteins from plants, algae, bacteria, krill, shrimps and other crayfish and crustacea by boiling the raw material one or more times in a liquor and subsequently filtering the extract from the boiling liquor, **characterised in that** the extracts of the filtering operation are allowed to cool, the extracts separating into three phases which are in turn separated by washing away the phase having the lowest specific gravity and decanting (or the like) the phase having an intermediate specific gravity from the phase having the highest specific gravity, the phase having the lowest specific gravity being neutralised by the addition of an acid, with the result that two liquid phases are formed which are separated and from which an oily phase containing astaxanthin esters is collected; the phase having the highest specific gravity being neutralised with an acid, and solids comprising astaxanthin and related carotenoids being filtered out and collected; and the phase having the intermediate specific gravity being neutralised and F⁻ ions and contaminants, especially heavy metal contaminants, being removed therefrom, the purified phase then being acidified, in a manner known per se, in order to precipitate proteins, after which the purified, precipitated proteins are collected by filtering or the like from the phase; in that the residue of the boiling operation is dried and subsequently dissolved in anhydrous formic acid or another strong acid and filtered, whereupon water is added to the filtrate with the result that the purified chitin is precipitated.

2. Procedure according to claim 1, **characterised in that** the phase having the intermediate specific gravity is purified by the addition of a train oil (fish oil), Ca⁺ ions, S²⁻ ions and (NH₄)₂HPO₄, for the precipitation of any F⁻ ions and contaminants, especially heavy metal contaminants, the precipitated material being removed from the solution by washing away or the like.

3. Procedure according to claim 1 or 2, **characterised in that** the boiling operation is carried out in two steps in the liquor which has a concentration of 1.0N of a strong alkaline solution.

## Revendications

1. Procédé de préparation d'astaxanthine et de carotinoïdes apparentées, d'esters d'astaxanthine, de chitine et de protéines, à partir de plantes, d'algues, de bactéries, de krills, de crevettes, de crabes et autres crustacés, comprenant une ou plusieurs cuissons de la matière première dans une solution alcaline et la filtration consécutive des extraits à partir de la solution en ébullition, **caractérisé par** le fait qu'on laisse refroidir les extraits de filtration, ces extraits se séparant alors en trois phases que l'on sépare par flottation la phase ayant le plus faible poids spécifique et décantation (ou par un procédé similaire), la phase ayant le poids spécifique intermédiaire, la phase ayant le poids spécifique le plus élevé, que l'on neutralise la phase qui présente le plus faible poids spécifique par addition d'un acide, ce qui entraîne la formation de deux phases liquides que l'on sépare et dont on tire une phase huileuse contenant un ester d'astaxanthine ; que l'on neutralise la phase qui présente le poids spécifique le plus élevé à l'aide d'un acide et sépare et recueille par filtration les substances solides constituées par de l'astaxanthine et des carotinoïdes apparentés ; et que l'on neutralise la phase qui présente le poids spécifique intermédiaire dont on élimine les ions F⁻ et les substances polluantes, notamment les métaux lourds, puis acidifie - de manière connue en soi - la phase purifiée pour produire la précipitation des protéines, ensuite, on recueille par filtration ou autre les protéines précipitées, purifiées, à partir de cette phase ; que l'on sèche le résidu de cuisson et le dissout ensuite, dans de l'acide formique anhydre ou dans un autre acide fort et filtre, puis ajoute de l'eau au filtrat, ce qui entraîne la précipitation de la chitine purifiée.

2. Procédé selon la revendication 1, **caractérisé par** le fait que la phase qui présente le poids spécifique intermédiaire est purifiée par addition d'huile de poissons, d'ions Ca⁺, d'ions S²⁻ et de (NH₄)₂HPO₄, pour la précipitation de certains ions F⁻ et de polluants, notamment de métaux lourds le produit précipité étant éliminé de la solution par flottation ou autre.

3. Procédé selon la revendication 1 ou 2, **caractérisé par** le fait que la cuisson est réalisée en deux étapes dans la solution alcaline qui présente une concentration de 1,0 N correspondant à une solution fortement alcaline.
